# EUROPEAN PATENT APPLICATION

(11) **EP 4 400 165 A2**
(43) Date of publication of application: **17.07.2024**
(21) Application number: 24171612.5
(22) Date of filing: 28.01.2016
(51) Int. Cl.: A61P 13/10

(54) **VAGINAL DRUG DELIVERY DEVICE**

(30) Priority: 30.01.2015 EP 15153322
(62) Divisional of application: 16701812.6
(71) Applicant: Li Galli B.V., 2514 AC Den Haag (NL)
(72) Inventor: DE LAAT, Wilhelmus Nicolaas Gerardus Maria, 2514 AC Den Haag (NL); VAN DER VAART, Carl Huibert, 2514 AC Den Haag (NL)
(74) Representative: van Someren, Petronella F. H. M.

(57) **Abstract**

The present invention relates to a vaginal drug delivery device configured for placement within the vagina, comprising a housing, at least one reservoir comprising a biologically active compound that is not normally administered via the vagina and means for the controlled release of the compound from the reservoir into the vagina. Suitably the vaginal device further comprising means for gathering physiological data of a person carrying the device in her vagina. Alternatively, the vaginal drug delivery device configured for placement within the vagina, comprises a housing and means for gathering physiological data of a person carrying the device in her vagina.

## Description

### FIELD OF THE INVENTION

The present invention relates to a vaginal drug delivery device for use in the treatment of various medical conditions in women. The device enables "personalized" or "individualized medicine".

### BACKGROUND OF THE INVENTION

Vaginal ring systems are usually used for contraception and hormone replacement therapy. Different ring systems for these applications are known in the prior art.

### SUMMARY OF THE INVENTION

According to the invention it has now been found that other than hormonal drugs can also be administered and absorbed via the vagina. Administration via the vagina is in particular useful for compounds that have relatively low or negligible oral bio-availability, e.g. due to insufficient absorption, degradation in the gastro-intestinal (GI) tract (e.g. peptides and proteins), or other factors, for compounds with unwanted GI side-effects, for compounds with a narrow therapeutic range, for compounds with a short half-life (t½) that require frequent or high dosing, for compounds that require a complex temporal and/or dosing schedule for adequate efficacy, etc. Such compounds can be over-the-counter (OTC) compounds as well as prescription drugs.

The invention thus relates to a vaginal drug delivery device configured for placement within the vagina, comprising a housing, at least one reservoir comprising a biologically active compound that is not normally administered via the vagina and means for the controlled release of the compound from the reservoir into the vagina.

In another embodiment, the invention relates to a vaginal drug delivery device configured for placement within the vagina, comprising a housing and means for gathering diagnostic data of a person carrying the device in her vagina. In this embodiment, the device is strictly diagnostic.

In a further embodiment, means for drug release and diagnosis are combined in the ring. Release of the compound from the ring is preferably controlled in response to diagnostic data of the user of the ring gathered by measuring means that are preferably located in, - or connected to, - the ring. For this, the device further comprises means for gathering data of a person carrying the device in her vagina. The means for the controlled release of the compound from the reservoir are then configured - either via handheld device / mobile device or independently/interactively by the ring itself -to act in response to physiological data of a person carrying the device in her vagina.

The means for the controlled release of the compound from the reservoir in the vaginal device of the invention can also be pre-programmed with a defined release pattern, which is for example dependent on the compound to be dispensed. The defined release pattern comprises for example continuous release, pulsatile release, spiked release, timed release, intermittent release, or combinations thereof or any other desired release pattern. In a further embodiment, the release of the medicament can be "on demand". This means for example that amounts of medication can be added by the patient, based on her medical complaints or in response to results of her diagnostic sensor. This "on-demand" possibility can be combined with any of the aforementioned release patterns.

The means for gathering physiological data of a person carrying the device in her vagina may comprise but are not limited to means for measuring temperature, glucose levels in the transudate of the vaginal mucosa, physico-chemical properties of the cervical mucus (like viscosity, clarity quantity and moisture), means for monitoring hormone levels,-either in the cervical mucus or in the transudate of the vaginal mucosa-, in particular of the hormones FSH, LH, 17-β-estradiol and progesterone or combinations thereof, means for analyzing proteins or nucleic acids, such as DNA, means for detecting molecules, in particular signaling molecules, such as cytokines, means to monitor the (an)ovulatory cycle, means for measurement of hyaluronic acid, or combinations thereof.

In one embodiment, the invention relates to the vaginal device, which is loaded with the biologically active compound. The compound is then considered an integral part of the device.

Administration via a vaginal device according to the invention is in particular suitable for compounds that have relatively low or negligible oral bio-availability, unwanted side-effects in the gastrointestinal tract, a narrow therapeutic range and/or an efficacy that depends on a complex temporal administration schedule or a short half-life. Such compounds can be from various therapeutic classes, including, but not limited to, psychiatric and neurological drugs (e.g. escitalopram, haloperidol, pramipexole), compounds for the treatment of drug dependence (e.g. nicotine, naltrexone, buprenorphine), analgesics (e.g. morphine, buprenorphine,), hormones (e.g. gonadorelin, insulin, estrogens, progestogens), cardiovascular drugs (e.g. clonidine) and anticholinergics (e.g. oxybutynin, solifenacin). Compounds that are released vaginally and are close to the target organ (like vagina, bladder, uterus and ovaries) will exert a "local" effect by simple diffusion and thus higher concentrations can be obtained, even with a lower dose. The administration approach of the present invention is thus particularly suitable for such compounds.

The present invention is in particular useful for oxybutynin, escitalopram, pramipexole, buprenorphine, clonidine, gonadorelin, insulin, nicotine, progestogens, SPRMs. In addition to these compounds, their analogs, homologs or agonists thereof or related compounds from the same chemical class and having the same pharmaceutical effect can be administered via a vaginal ring.

### DETAILED DESCRIPTION

The invention thus relates to a new means of administration of certain biologically active compounds, in particular drugs, via a device that is placed in the vagina and from which the compound is released in a controlled manner. The compounds are drugs and other compounds that are usually not administered via the vagina.

The device comprises a housing in which the various components needed for the desired function are located. The housing can at the same time be the vehicle that is placed in the vagina. The device can have various functions but will in any case comprise a reservoir for the biologically active compound and means for dispensing the compound into the vagina and means for doing so in a controlled manner. Additional functions comprise for example means that measure certain parameters in the vagina, such as temperature, glucose, medication levels,hormone levels, physico-chemical parameters of the cervical mucus etc.

The device can be a ring or any other form that can comfortably be inserted into and carried in the vagina. The device can thus have different forms and functions, next to serving as a vehicle that assures continued presence within the vagina. The device may for example be a ring that has a flexible part and a rigid part, preferably in the top portion of the ring. The device is configured in such a way that the thicker rigid part can be compressed and will be inserted first, the ring will then defold after insertion with the rigid part settling smoothly in the deepest part of the vagina (fornix posterior) and the outer parts of the ring in flexible contact with the vaginal wall to prevent expulsion and enhance absorption.

In one embodiment, the ring comprises a positioning member configured for placement within the vagina and a device comprising one or more components selected from drug reservoirs, diagnostic means and also means for dispensing the drug from a reservoir into the vagina, wherein according to the invention the drug reservoir is loaded with a biologically active compound, in particular a compound of the type listed above. The compound, in particular a medicament, is preferably formulated such that it will be sufficiently stable during storage and the period that the ring is in place and can be formulated with an absorption enhancer, where necessary.

An important feature of the invention is that the release of the biologically active compound from the device can be controlled, even during presence of the device in the vagina, i.e. remotely. To achieve this the vaginal ring of the invention preferably has electronic equipment to program any schedule of release of medication. This allows for "personalized" or "individualized medicine". Schedules like cyclical, daily, nightly, hourly, continuously, or pulsed/ in spikes, on demand etc. can be programmed adapted to the user's needs.

The schedule can be pre-programmed and/or adjusted either remotely, based on wishes of the user (e.g. pain relief) or by the patient, a physician or nurse based on results of diagnostic tests transmitted to the outside world by the device, or by a direct adaptation in the release pattern by the device itself based on diagnostic measurements made by the device. The device is than suitably programmed such that a particular result of a measurement automatically leads to a corresponding adaptation of the release pattern of the compound. To achieve this, the device comprises measuring means, such as sensors, probes, etc. Suitable sensors are temperature sensors, pH sensors, glucose sensors or sensors that can measure physic-chemical properties of the cervical mucus ( viscosity, quantity, moisture, clarity) or biosensors for measuring biological compounds such as hormones or medication. In addition, information is stored in the device or can be sent to the device that instructs the device how to adapt the release in response to a measured parameter.

The invention can be used with any form of ring, but especially the embodiment described in PCT/EP2014/054148 and co-pending application EP-15188465 (not yet published). In another embodiment, the ring can have a ring shape, wherein the cross section of the ring is variable along the circumference of the ring. In one embodiment the ring, can be used in combination with the Intellicap^{™} technology as described in WO2011/039680. The Intellicap^{™} can be suitably used for harboring the release, control and diagnostic functions of the vaginal device.

The vaginal device of the invention contains a reservoir or a cartridge which can be filled with medicines that are absorbed vaginally at biologically active levels, either in the presence or the absence of an absorption enhancer or administered for vaginal topical drug delivery via programmable (either pre-programmed or by remote control) release.

The present invention is based on the realization that other compounds than hormones can be administered via a vaginal device. The invention thus relates to use of the device for the administration of biologically active compounds, such as drugs, that are not normally administered vaginally for treatment of disorders and conditions for which these compounds are normally used. The difference is the administration route.

The systemic drug delivery via the vagina has many general advantages as compared to oral administration, such as no nausea (unless the administered drug also causes nausea if administered systemically, like e.g. cytostatics), no irritation of the gastrointestinal tract, elimination of hepatic first pass drug metabolism, prevention of enzymatic degradation of drugs in the gastrointestinal tract. As compared to parenteral administration the ring is less invasive. It is an excellent system for long term medication and has the advantages of high bioavailability enabling lower doses for drugs which are currently administered orally, high bioavailability, thus avoiding parenteral delivery as injection, infusion or subcutaneous delivery, and has the added advantage of self-empowerment by enabling self-administration, self-control of insertion and removal, leading to high convenience and high compliance. Obviously the user remains ambulatory and outpatient.

The present invention offers unique advantages in the treatment of diseases and/or situations that require more frequent and/or untimely and/or complex drug delivery schedules compared to separate single oral doses or separate single injections.

The present invention offers in particular excellent medication regulation for diseases that require stable drug serum levels for adequate disease control (e.g. hypertension, depression, psychosis and diabetes) because the release from the device can be adapted to the need of the user at a particular moment.

The present invention also allows for pulsatile delivery of compounds. In many physiological states of the female biological systems, several substances (like pituitary hormones and their pharmacological (ant)agonists, but also TSH, etc.) are released in a pulsatile way and therefore certain diseases can only be treated by administering the medicine on a pulsatile way, for example for ovulation induction in Kallman syndrome. The possibility to program the vaginal device for pulsatile release is then very useful.

The present invention also shows specific advantages for treatment of diseases that require a high compliance rate in drug intake. An inserted and activated the ring will have a 100% compliance rate during the stay in the vagina, which can be 1-4 weeks.

The present invention allows for drug delivery of drugs that are currently not used for certain indications because they require daily injections or an intravenous line, e.g. the Selective Progesterone Receptor Modulators (SPRMs) for the indication of contraception.

The present invention is perfectly suited for drug delivery at untimely hours (such as nightly), for pulsed drug delivery, such as treatment of anovulation due to hypogonadotropic hypo-estrogenic amenorrhea, like the Kallman syndrome, with pulsed GnRH in patients with a wish to have children, and such as the treatment of OAB.

Various drug delivery regimes can also be combined. The device may e.g. contain multiple reservoirs for different medicaments. Another example of combined delivery is pulsed drug delivery on demand or preprogrammed, on top of a constant or steadily declining baseline level, e.g. pain relief and therapy, nicotine addiction and OAB.

In preferred embodiments, the device is for use in the support of smoking cessation and comprises nicotine with a programmed delivery profile. For use in the treatment of mild cognitive impairment the device comprises nicotine and is for example programmed for continuous release. For use in the treatment of hypertension in non-compliant patients the device is loaded with clonidine. For use in the treatment of diabetic neuropathic pain the device also comprises clonidine and is programmed for continuous release.

The present invention also allows for drug delivery close to the anatomically adjacent organs like the uterus, the vagina and the bladder. One example is a vaginal device, such as a ring, with a progestogen for contraception and/or menometrorrhagia to replace the known uterine device Mirena^{®}. Such a ring, unlike the Mirena^{®}, allows for a continuously adequate serum level of progestogen over time and has programmable dose adjustment if required. Another example is medication for OAB or endometriosis or medication for contraception, if necessary combined with anti-HIV medication in certain subjects.

In a particularly preferred embodiment, the device of the invention is used for treating overactive bladder (OAB) or urge incontinence with oxybutynin or another anticholinergic compound. Oral anticholinergics have a short t½, and a strong first-pass effect. They must be frequently and/or highly dosed and oxybutynin's primary metabolite, N-desethyloxybutynin (DEO), causes strong side-effects, particularly dry mouth, after oral administration. When oxybutynin (or another similar compound) is administered by means of a vaginal drug delivery device according to the invention, the therapeutic window of oxybutynin is widened by improving the efficacy/side-effect ratio, by lowering the side-effects via avoiding the first-pass effect and thus lowering the metabolite level, by improving the efficacy by allowing use of a higher dose, by benefiting from the "local" diffusion effect close to the bladder and by benefiting from the possibility to program the device for pulsatile delivery of the anticholinergic, in particular oxybutynin. Continuous dosing of antagonists will lead to upregulation of the G-coupled receptor, thus decreasing the efficacy. Upregulation will be avoided by pulsatile administration.

The invention thus relates to oxybutynin for use in the treatment of urinary and bladder problems, in particular urge incontinence, wherein oxybutynin is administered to a person in need of treatment via the vagina, in particular by means of a vaginal device, more in particular the vaginal device as disclosed herein. The invention also relates to the use of oxybutynin for the preparation of a medicament for the treatment of urinary and bladder problems, in particular urge incontinence, wherein the medicament is formulated as a vaginal device comprising oxybutynin or a related compound. The invention also relates to a method for the treatment of urinary and bladder problems, in particular urge incontinence, wherein oxybutynin or a related compound is administered to a person in need of treatment via the vagina, in particular by means of a vaginal device, more in particular the vaginal device as disclosed herein. In particular, the oxybutynin or related compound is released from the device in a pulsatile release pattern.

The oxybutynin, or any other anticholinergic, like solfenacin, is suitably in the form of a liquid which is able to dispense the drug from the vaginal drug delivery system to the vaginal cavity. For this the active ingredient (oxybutynin) should be locally in solution or must be capable of being solved when administered as a suspension. In order to facilitate release from the device by penetrating the membrane adjustments tot he pH can be made or a solubilizing agent, such as a cyclodextrin derivative, can be added. In one embodiment the oxybutynin containing liquid contains oxybutynin base, hydrochloric acid salt, or any other biocompatible salt, crystal, co-crystal or complex or mixtures thereof, such as sulphate, phosphate, complexes with cyclodextrin, for example hydroxypropyl-beta-cyclodextrin.

The oxybutynin containing liquid can be a true solution, a colloidal solution, an emulsion, a micro-emulsion, a nano-emulsion, a suspension, a micro-suspension or a nano-suspension. The oxybutynin containing liquid can be a Newtonian or non-Newtonian liquid of low to medium/high viscosity. The characteristics of the device determine the maximum viscosity that can still be pumped from the vaginal drug delivery system.

The oxybutynin containing liquid suitably comprises aqueous or non-aqueous biocompatible solvents or mixtures thereof. It suitably comprises a high concentration of active substance allowing optimal drug absorption from the vaginal cavity, such as higher than 80 mg/ml.

The oxybutynin containing liquid is formulated such that no drug precipitation occurs in the vaginal environment. This can for example be prevented by adjusting the pH and/or adding a small amount of polyethylene glycol of propylene glycol.

The oxybutynin containing liquid is preferably stable for at least 12 months under the drug product storage condition and at least stable for 1 month at body temperature.

In the context of oxybutynin the device can include the function of measuring electromyography (EMG) signals of the bladder. These signals can predict early bladder contractions and the device can subsequently release oxybutynin "on demand".

The invention also relates to escitalopram for use in the treatment of depression, wherein escitalopram is administered by means of a vaginal device.

In a further embodiment the invention relates to pramipexole for use in the treatment of Parkinson's disease, wherein pramipexole is administered to a person in need of treatment by means of a vaginal device.

Another embodiment relates to buprenorphine for use in the treatment of opioid addiction or chronic pain, wherein buprenorphine is administered to a person in need of treatment by means of a vaginal device.

The invention also relates to clonidine for use in the treatment of neuropathic pain or hypertension, wherein clonidine is administered to a person in need of treatment by means of a vaginal device.

The invention also relates to the use of escitalopram, pramipexole, buprenorphine and clonidine for their respective medical indications, wherein the compound is administered to a person in need of treatment via the vagina, in particular by means of a vaginal device, more in particular the vaginal device as disclosed herein. The invention further provides methods for treating these indications wherein the compound is administered to a person in need of treatment via the vagina, in particular by means of a vaginal device, more in particular the vaginal device as disclosed herein.

In a preferred embodiment, the device of the invention is used for addressing the hypogonadotropic-hypoestrogenic state in females (such as in Kallman syndrome, also anorexia) where pulsatile administration (one pulse every 90 minutes) of GnRH is required to have the ovaries starting to produce female hormones and induce an ovulatory status if a pregnancy wish exists. Those women can achieve a biologically normal ovulatory status with a GnRH ring, also during the time they have no wish to have children. Currently those patients are substituted with contraceptives for their hypo-hypo hormonal status for the period(s) that no child-wish exists. For this period substitution with oral contraceptive hormones is the current practice, but this not sufficient to prevent medical complaints and diseases (like osteoporosis). Those women could achieve a biologically normal ovulatory status with a GnRH ring, also during the time they have no wish to have children, thus avoiding the medical complaints and problems.

The invention thus relates to gonadorelin for use in the treatment of Kallman syndrome, wherein gonadorelin is administered to a person in need of treatment via the vagina, in particular by means of a vaginal device, more in particular the vaginal device as disclosed herein. The invention also relates to the use of gonadorelin for the preparation of a medicament for the treatment of Kallman syndrome, wherein the medicament is formulated as a vaginal device comprising gonadorelin or a related compound. The invention also relates to a method for the treatment of Kallman syndrome, wherein gonadorelin or a related compound is administered to a person in need of treatment via the vagina, in particular by means of a vaginal device, more in particular the vaginal device as disclosed herein. In particular, the gonadorelin or related compound is released from the device in a pulsatile release pattern.

In an another preferred embodiment the device is used for treating diabetes with insulin or insulin agonists. The insulin can be administered in preprogrammed dose/quantity/time schedules. The insulin administration can be superimposed by certain doses of insulin "on-demand", based on subjective medical symptoms of hyperglycemia and/or results of the diagnostic glucose sensoring. Either after reading the results on the computer/handheld mobile device or independently and interactively via an algorithm in the device itself. The insulin can be administered in solution, with or without an absorption enhancer
In another preferred embodiment, the device of the invention is used for nicotine replacement therapy (NRT) for smoking cessation. An important biological factor in smoking addiction is the nicotine addiction. Current pharmacotherapies (such as patches, gums, sprays) show very low success rates of around 10% (after 6 months) or lower. The constant nicotine administration via the current patches occupies the nicotine receptors continuously, leading to desensitization of those receptors, while spray and gum as well as the current patches also fail to result in the spike-levels of nicotine, which occur in smoking itself. With the device of the invention the nicotine-release can be programmed such that it mimics smoking-induced release of nicotine as much as possible with regard to dose, interval and "speed", i.e. the time needed for nicotine to appear in the brain after administration. It should subsequently be tapered (with longer intervals and smaller doses). This results in repetitive and rapid spikes with a level of serum nicotine as in smoking itself, and a gradual diminishing of dose, together with longer intervals. Experts agree that such a smoking-mimicking release pattern would be ideal to support smoking cessation, while the nicotine "reward" is steadily diminished with respect to absolute dose as time intervals to support the gradual cure of the nicotine addiction status.

For this application, the ring of the invention is programmed to deliver nicotine vaginally in a way that differs from the current patches in several ways, namely the absolute nicotine level is adjusted to mimic the nicotine levels of smokers, the nicotine schedule is programmed to mimic smoking frequency. Subsequently the nicotine levels are gradually diminished ("tapered") over for instance 3 monthly cycles, and simultaneously the nicotine intervals are gradually lengthened over those cycles The pulses/intervals are combined with a certain circadian rhythm of a basal low nicotine level for certain hours at certain times (as between 4:00 and 8:00 AM), the low levels in many addicted persons, during a time that smoking and the use of nicotine spray or gums are difficult. The schedule can be user-controlled, or not. If pre-programmed it delivers this consistent and gradually decreasing and diminishing pattern ("tapering") that cannot be forgotten and/or tampered with (as is the case with patches, gums and spray). The device of the invention can function for 3 weeks without any action demanded from the user.

The invention thus relates to nicotine for use in the support of smoking cessation or in the treatment of mild cognitive impairment, wherein nicotine is administered to a person in need of support or treatment by means of a vaginal device, more in particular the vaginal device as disclosed herein. The invention also relates to the use of nicotine for the preparation of a medicament for support of smoking cessation, wherein the medicament is formulated as a vaginal device comprising nicotine or a related compound. The invention also relates to a method for supporting smoking cessation, wherein nicotine or a related compound is administered to a person in need of treatment via the vagina, in particular by means of a vaginal device, more in particular the vaginal device as disclosed herein. The nicotine is preferably released from the device into the vagina in a tapered release pattern as defined above.

In another embodiment the ring of the invention can be used to treat people suffering from sleeping problems like insomnia, which is not necessarily a problem with falling asleep, but with staying asleep. They would require a continuous low dose of sleeping medication or a repeated dose during the first hours of sleep in order to stay asleep which can only be done with the vaginal device of the invention without the female waking up to take the medication. The sleeping medication is then used for treating sleeping disorders, wherein the medication is administered vaginally, preferably via a vaginal device, more preferably the vaginal device disclosed herein.

In a further embodiment the device is used for providing pain relief, with drug delivery on demand. Patients that require parenteral pain relief medication have a pump and a mechanism to adjust the dose. The pump makes them non-ambulatory. The vaginal device disclosed herein solves that problem, leading to better efficacy and a significant improvement of the quality of life of the patient.

In addition to the above, the device can be used for topical drug delivery. Topical drug delivery via a vaginal device has many general advantages, in particular reduced side effects from reducing or eliminating systemic exposure and reduced dose by delivery to site of action.

The active ingredient contained in the vaginal device, is suitably in the form of a liquid which is able to dispense the drug from the vaginal drug delivery system to the vaginal cavity. For this the active ingredient should be locally in solution or must be capable of being solved when administered as a suspension. In order to facilitate release from the device by penetrating the membrane adjustments tot he pH can be made or a solubilizing agent, such as a cyclodextrin derivative, can be added. In one embodiment the liquid contains the base of the active ingredient, hydrochloric acid salt, or any other biocompatible salt, crystal, co-crystal or complex or mixtures thereof, such as sulphate, phosphate, complexes with cyclodextrin, for example hydroxypropyl-beta-cyclodextrin.

The liquid containing the active ingredient can be a true solution, a colloidal solution, an emulsion, a micro-emulsion, a nano-emulsion, a suspension, a micro-suspension or a nano-suspension. The said liquid can be a Newtonian or non-Newtonian liquid of low to medium/high viscosity. The characteristics of the device determine the maximum viscosity that can still be pumped from the vaginal drug delivery system.

The liquid containing the active ingredient suitably comprises aqueous or non-aqueous biocompatible solvents or mixtures thereof. It suitably comprises a high concentration of active substance allowing optimal drug absorption from the vaginal cavity, such as higher than 80 mg/ml.

The liquid containing the active ingredient is formulated such that no drug precipitation occurs in the vaginal environment. This can for example be prevented by adjusting the pH and/or adding a small amount of polyethylene glycol of propylene glycol.

The liquid containing the active ingredient is preferably stable for at least 12 months under the drug product storage condition and at least stable for 1 month at body temperature.

The device that is used according to the invention can also be used for diagnostic purposes. In a first embodiment, the diagnostic data is used to adapt the release of the compound or compounds from the device. However, it is also possible that the diagnostic function is the only function of the device. In that case, the device does not necessarily comprise a drug reservoir.

A preferred feature of the diagnostic part of the vaginal ring invention is monitoring of the female (an)ovulatory cycle. In a normal ovulatory cycle of the women there will be changes in a couple of female (pre)hormones, indicating the (approaching) event of the ovulation. The temperature is in reality only a signal of the past ovulation and as such less valuable, but may be used in combination with for example chemical monitoring to improve prediction and/or monitoring. Ovulation detection/cycle monitoring, can be useful in all females with fertility problems who want to know their ovulation time, for cycle and/or stimulation monitoring in ART cycles (Assisted Reproduction Techniques), like artificial insemination, IVF, etc or in females who want to exercise a "natural" contraception and thus need to know the risk-free/intercourse safe period.

Examples of diagnostic features of the ring of the invention are in particular temperature monitoring, monitoring of glucose levels, monitoring of various steroid hormones (17-β-estradiol, progesterone) and releasing hormones (FSH, LH, TSH etc.), measuring medication levels measuring pH or physico-chemical properties of the cervical mucus, protein analysis, DNA compounds analysis, detecting signaling molecules (e.g. cytokines and others). The ring is in contact with the vaginal mucosa and the measurements with the sensors are either made in the transudate of the vaginal wall and/or the cervical mucus.

Certain parameters measured in the vagina can be used to calculate the level of the parameter in serum. For this a conversion has to be made. The skilled person knows how to do this.

Another function that can be included in the device are measurements of the physico-chemical properties of the cervical mucus (like quantity, moisture, clarity and viscosity) that change during the female cycle. At ovulation the mucus is most transparent. This function can thus be used to predict the approach of ovulation.

In order to determine whether a woman is compliant in wearing the device a temperature sensor can be included. If the device is located in the vagina the temperature will record about 38°C and these results can be recorded and transmitted, either to an outside computer or handheld device. In addition, the device can also keep a log of the amount of compound that is released, if applicable.

The present invention can be performed with any vaginal device. In principle the device can have any shape. In a preferred embodiment, though, the device is a ring, in particular a ring which contains a flexible part and a rigid part. In this embodiment, the flexible part of the ring has several proposed shapes and may also contain an antenna for communication with the ring, e.g. related to programming, or for transmission of data, generated by the diagnostic systems in the ring to the outside and/or for receiving commands from outside the body. The electronics are preferably based on the Intellicap^{™} technology as described in WO2011/039680. Furthermore, the ring contains a special pump and container/cartridge and communication and data transfer means.

The ring of the invention is designed in such a way that it is safe, causes no lesion to the vaginal epithelium and cervix at insertion, removal or use of the device, is produced from material(s) of approved medical grade, is non-irritating, non-allergenic, has no risk of breakage, no risk of disintegration of electronic parts into the vagina, and comprises a built-in prevention of erroneous dose delivery.

The outer surface of the ring is suitably smooth to avoid irritation of the vaginal wall. The inner surface of the ring is preferably smooth to allow convenient intercourse. The elastic property of the ring offers a stabile positioning of the ring in the vagina. Suitably, the electronic parts with actuator are connected to the medication reservoir and housed together within the ring without compromising the round or oval structure of the ring. The configuration of the ring is such that the correct insertion is unequivocal.

The ring that is used in the invention can be pre-programmed for personalized medication or programmed and program-adjusted from a personal external transmitter device using Near Field Communication (NFC) technology, Bluetooth or other systems. In addition, the ring can be configured such that data gathered by the diagnostic means in the ring is directly translated into an adaptation of the release parameters.

The ring can collect data on the administered medication for evaluation by the physician. Also diagnostic data can be transmitted and stored when sensors are used in the ring. Electronics and mechanics suitably form a rigid and thicker portion of the ring that will smoothly settle in the deeper part of the vagina (fornix posterior) after insertion.

The diagnostic results can be communicated with an outside transmitter device like computer, smart phone or network, or used in autonomous feed-back mode to trigger release from the medication reservoir.

Other preferred features of the ring of the invention are related to ovulation prediction/monitoring and include the assay of four hormones: FSH, LH, 17-β-estradiol and progesterone or combinations thereof, measurement of hyaluronic acid.

The possibility of a "lab-on-a-chip", which includes drug assays to establish the optimal serum levels of medications mentioned herein, but also of anti-depressants, anti-thrombotics, (etc.), as well as a glucose sensor, will allow remotely controlled adjustment of the medication or the interaction of diagnostic and drug delivery parts of the ring.

In a preferred embodiment all functionalities of the drug delivery part and diagnostic part are therefore combined to provide an independently and interactive system that can adjust the drug delivery dose based on the results of the diagnostics.

In one embodiment, the ring can interactively and continuously monitor the glucose levels in a diabetic female and adjust the insulin delivery accordingly. In addition, the results of the monitoring can be sent to the outside world, for example to a handheld device such as a smartphone or tablet. The user can then also monitor her glucose levels. A suitable sensor for glucose monitoring in the vagina is a CGS (continuous glucose sensoring) sensor.

In another preferred embodiment the diagnostic part can predict an upcoming epileptic insult and deliver a proper dose of indicated medicine to prevent this upcoming epileptic insult.

For drug delivery, the ring comprises one or more components selected from a drug storage space (reservoir), means for regulated drug release, a transport system to the drug release site, a membrane or micro-pore surface at the drug delivery site, means for variable, continuous, interrupted or single drug delivery, means for drug delivery in fluid solution, in (adhesive) gel composition, in a foam composition, in a fluid composition to restore or protect vaginal ecology (e.g. pH) etc.

The functional components can suitably be integrated in an electronic capsule. Such electronic capsule can for example comprise one or more of the following elements: an internal connection electronic system to diagnostic sensors, an internal connection to drug release parts (storage, transport, contact surface), means for analysis of diagnostic parameters, means for storage of diagnostic data, means for data transmission to external data equipment, a receiver for external control of function, preferably embedded in a protected housing, alert signaling at malfunction, automatically switch off at malfunction etc.

For optimizing the conditions for use, the ring is configured such that it is in place intra-vaginally for 1-4 weeks, that intercourse remains possible at use, that no lesions of the partner occur at intercourse, that there is no adverse effect of the drug delivered by the device on the partner, that there is no expulsion of the device and no induced or involuntary discharge of the active compound, that the position of the device in the vagina allows correct monitoring, etc.

The ring is especially accepted by the female because of self-empowerment of use, by clear instructions, by self-insertion and self removal, by comfort of use (no pain or irritation) during 1-4 weeks, by the partner's acceptance and non-interference during coitus, and by clear (pre-programmed) use or easy adjustment by tele-command.

The invention also relates to a vaginal ring comprising a positioning member configured for placement within the vagina and an electronic device comprising one or more components selected from drug reservoirs, diagnostic systems and systems for dispensing the drug from a reservoir into the vagina, wherein the drug reservoir is loaded with a medicament and the medicament is preferably formulated such that it will be sufficiently stable during storage and the period that the ring is in place and is optionally formulated with an absorption enhancer. The compound has relatively low or negligible oral bio-availability, e.g. due to insufficient absorption, degradation in the gastro-intestinal (GI) tract (e.g. peptides and proteins), or other factors, or unwanted GI side-effects, a narrow therapeutic range, requires for efficacy a complex temporal administration schedule.

Administration of biologically active compounds via a vaginal device according to the invention allows for any administration schedule. In contrast to the known vaginal rings used for the administration of hormones, in which the compound is contained in a polymeric matrix and released therefrom in a manner dictated by the location and the concentration of the compound and the polymer, the presence of a reservoir in the ring device allows administration of any dosage. Moreover, even during the presence of the device in the vagina the dosage and release pattern can be adapted, for example in response to measurements made in the vagina made by diagnostic means in the device.

In the present application the words "device" and "ring" are used interchangeably.

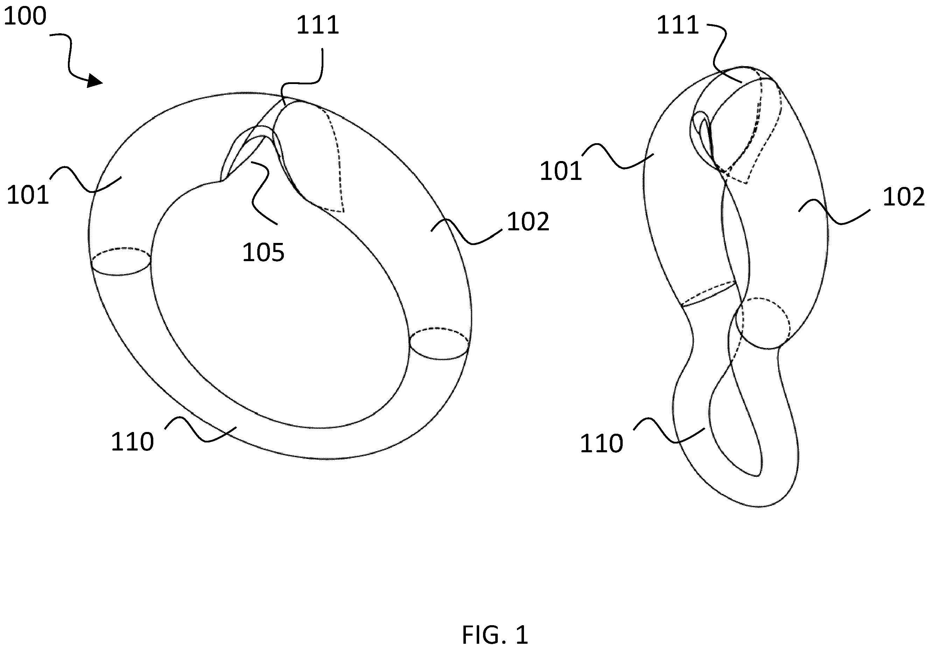

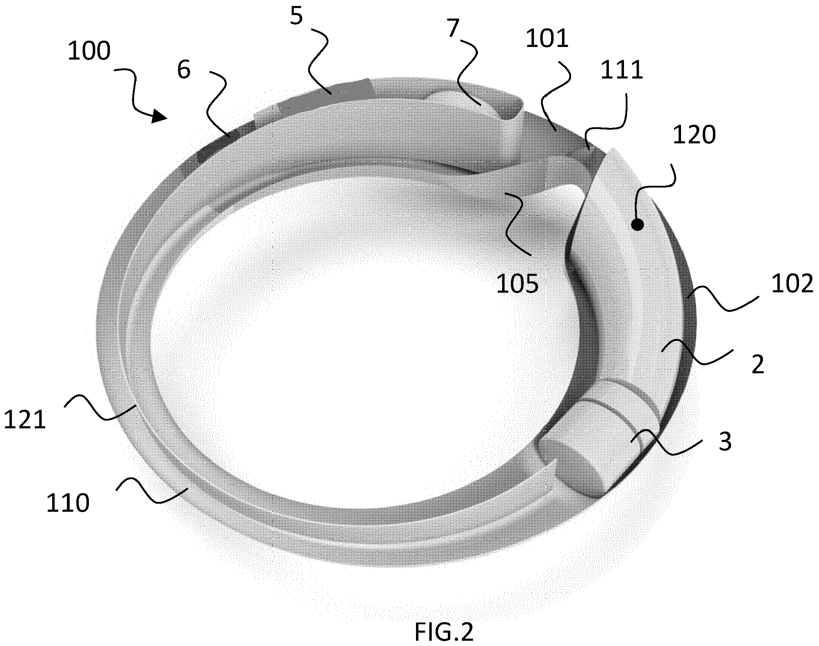

The present invention will now be further illustrated in the Examples that follow. The Examples are given for illustration purposes only and are not intended to limit the invention in any way. The experiments described in the Examples were conducted with the device shown in Figures 1 and 2.

Figure 1 illustrates the shape of the device used in accordance with the present invention in an extended (left) and collapsed state (right). This shape comprises a first rigid member 101, a second rigid member 102, a first flexible member 111, and a flexible part 110. Here, first flexible member 111 is made of an elastic material and comprises a recess 105 to allow first and second rigid member 101, 102 to move towards each other when device 100 is squeezed into the collapsed state.

The functional parts of the drug delivery and/or diagnostic mechanism are incorporated into the rigid members 101, 102, whereas the (electrical) interconnect can be accommodated in the flexible member 111 or flexible part 110.

Figure 2 illustrates the device of figure 1 showing an exemplary arrangement of some of the components of the device. Here, it can be seen that the functional parts of the drug delivery mechanism, i.e. battery 7, controller 6, pump 3, reservoir 2, and sensor 5 are incorporated in first rigid member 101 and second rigid member 102. Moreover, an opening 120 can be identified through which the medicament held in reservoir 2 is pumped out by pump 3. This opening will be located at such a place on the outer perimeter of the ring to guarantee optimal contact with the vaginal mucosa. Furthermore, an electrical interconnect 121 can be seen that connects the various components to each other and to battery 7.

In figure 2, a transceiver may be incorporated in controller 6 or may alternatively be arranged as a separate component in first rigid member 101. This transceiver allows data communication between controller 6 and external systems or devices for remote control or for exchanging data such as measurement data.

First rigid member 101 is provided with an opening or membrane to enable sensor 5 to perform a predefined measurement. Similar openings or membranes can be provided if additional sensors are used which may or may not be arranged in first rigid member 101. These openings will be located at such places on the outer or inner perimeter of the ring to guarantee optimal contact with the vaginal mucosa or cervical mucus.

### CLAUSES

1. A vaginal drug delivery device configured for placement within the vagina, comprising a housing, at least one reservoir comprising a biologically active compound that is not normally administered via the vagina and means for the controlled release of the compound from the reservoir into the vagina.
2. Vaginal device according to clause 1, further comprising means for gathering physiological data of a person carrying the device in her vagina.
3. Vaginal device according to clause 1 or 2, wherein the means for the controlled release of the compound from the reservoir are configured to act in response to physiological data of a person carrying the device in her vagina.
4. Vaginal device according to clause 1 or 2, wherein the means for the controlled release of the compound from the reservoir are operated remotely, in particular by the person carrying the device in her vagina, a nurse or a physician.
5. Vaginal device according to clause 1 or 2, wherein the means for the controlled release of the compound from the reservoir are pre-programmed with a defined release pattern.
6. Vaginal device according to clause 4, wherein the defined release pattern comprises continuous release, pulsatile release, spiked release, timed release, intermittent release, or combinations thereof.
7. Vaginal device according to clause 6, wherein the release is on-demand, if necessary in combination with the release patterns in clause 6.
8. A vaginal drug delivery device configured for placement within the vagina, comprising a housing and means for gathering physiological data of a person carrying the device in her vagina.
9. Vaginal device according to any one of the clauses 2-7, wherein the means for gathering diagnostic data of a person carrying the device in her vagina comprise means for measuring temperature, glucose levels, physico-chemical properties of cervical mucus like clarity, moisture and viscosity, means for monitoring medication levels, means for measuring hormone levels, in particular of the hormones FSH, LH, 17-β-estradiol and progesterone or combinations thereof, means for analyzing proteins or nucleic acids, such as DNA, means for detecting molecules, in particular signaling molecules, such as cytokines, means to monitor the (an)ovulatory cycle, means for measurement of hyaluronic acid, or combinations thereof.
10. Vaginal device according to clause 9, wherein the measurements are made in the transudate of the vaginal mucosa and/or the cervical mucus.
11. Vaginal device according to any one of the clauses 1-10, which is loaded with the biologically active compound.
12. Vaginal device according to any one of clauses 1-11, wherein the compound is a compound that has relatively low or negligible oral bio-availability, short half life ( t ½),strong first -pass effect, unwanted side-effects in the gastrointestinal tract, a narrow therapeutic range and/or an efficacy that depends on a complex temporal administration schedule.
13. Vaginal device according to any one of the clauses 1-12, wherein the compound is selected from the group consisting of psychiatric and neurological drugs (e.g. escitalopram, haloperidol, pramipexole), compounds for the treatment of drug dependence (e.g. nicotine, naltrexone, buprenorphine), analgesics (e.g. morphine, buprenorphine,), hormones (e.g. gonadorelin, insulin, estrogens, progestogens), cardiovascular drugs (e.g. clonidine) and anticholinergics (e.g. oxybutynin, solifenacin).
14. Vaginal device according to any one of the clauses 1-13, wherein the compound is selected from the group consisting of oxybutynin, escitalopram, pramipexole, buprenorphine, clonidine, gonadorelin, insulin, nicotine, a progestogen, an SPRM, or analogs, homologs or agonists thereof or related compounds from the same chemical class and having the same pharmaceutical effect.
15. Oxybutynin for use in the treatment of urinary and bladder problems, in particular urge incontinence, wherein oxybutynin is administered to a person in need of treatment by means of the vaginal device according to any one of the clauses 1-14.
16. Escitalopram for use in the treatment of depression, wherein escitalopram is administered by means of the vaginal device according to any one of the clauses 1-14.
17. Pramipexole for use in the treatment of Parkinson's disease, wherein pramipaxole is administered to a person in need of treatment by means of the vaginal device according to any one of the clauses 1-14.
18. Buprenorphine for use in the treatment of opioid addiction or chronic pain, wherein buprenorphine is administered to a person in need of treatment by means of the vaginal device according to any one of the clauses 1-14.
19. Clonidine for use in the treatment of postmenopausal complaints or hypertension, wherein clonidine is administered to a person in need of treatment by means of the vaginal device according to any one of the clauses 1-14.
20. Gonadorelin for use in the treatment of the syndrome of Kallmann, wherein gonadorelin is administered to a person in need of treatment by means of the vaginal device according to any one of the clauses 1-14.
21. Insulin for use in the treatment of diabetes, wherein insulin is administered to a person in need of treatment by means of the vaginal device according to any one of the clauses 1-14.
22. Nicotine, for use in the support of smoking cessation or in the treatment of mild cognitive impairment, wherein nicotine is administered to a person in need of support or treatment by means of the vaginal device according to any one of the clauses 1-14.
23. Progestogen for use in contraception, wherein progestogen is administered to a person in need of treatment by means of the vaginal device according to any one of the clauses 1-14.
24. An SPRM for use in contraception, wherein the SPRM is administered to a person in need of treatment by means of the vaginal device according to any one of the clauses 1-14.

## Claims

1. A vaginal drug delivery device configured for placement within the vagina, comprising a housing, at least one reservoir comprising a biologically active compound that is not normally administered via the vagina, dispensing means and means for the controlled release of the anticholinergic compound from the reservoir into the vagina,
wherein the controlled release is programmable according to a defined release pattern.

2. Vaginal device as claimed in claim 1, wherein the device is a ring which comprises a first rigid member (101), a second rigid member (102), a first flexible member (111) and a flexible part (110), wherein the first flexible member is made of an elastic material and comprises a recess (105) to allow the first and second rigid members (101, 102) to move towards each other when the device (100) is squeezed from the extended into the collapsed state.

3. Vaginal device as claimed in claim 1 or 2, wherein the defined release pattern is pre-programmed.

4. Vaginal device as claimed in any one of claims 1 to 3, wherein the defined release pattern is selected from pulsatile release, spiked release, timed release, intermittent release, or combinations thereof.

5. Vaginal device as claimed in any one of the claims 1 to 4, wherein the release is according to a pre-programmed release schedule which is selected from cyclical, daily, nightly, hourly.

6. Vaginal device as claimed in any one of the claims 1 to 5, wherein the defined release pattern is on top of a constant or steadily declining baseline level.

7. Vagina device as claimed in any one of the claims 1 to 6, wherein the release is adjustable to the user's needs.

8. Vaginal device as claimed in any one of the claims 1 to 7, wherein the means for the controlled release of the compound from the reservoir are operated remotely.

9. Vaginal device as claimed in claim 8, wherein the means for the controlled release of the compound from the reservoir are operated remotely based on the wishes of the user.

10. Vaginal device as claimed in any one of the claims 1 to 9, wherein the release is on demand.

11. A vaginal drug delivery device as claimed in any one of the claims 1 to 10, further comprising means for gathering physiological data of a person carrying the device in her vagina.

12. Vaginal device as claimed in claim 11, wherein the means for the controlled release of the compound from the reservoir are operated remotely based on results of diagnostic tests transmitted to the outside world by the device.

13. Vaginal device as claimed in claim 12, wherein the means for the controlled release of the compound from the reservoir are operated by the person carrying the device in her vagina, a nurse or a physician.

14. Vaginal device as claimed in any one of the claims 1 to 13, wherein the adjustment of the release is done from a personal external transmitter device, in particular using Near Field Communication technology or Bluetooth.

15. Vaginal device as claimed in any one of the claims 1 to 14, wherein the biologically active compound that is not normally administered via the vagina is selected from the group consisting of psychiatric and neurological drugs (e.g. escitalopram, haloperidol, pramipexole), compounds for the treatment of drug dependence (e.g. nicotine, naltrexone, buprenorphine), analgesics (e.g. morphine, buprenorphine,), hormones (e.g. gonadorelin, insulin, estrogens, progestogens), cardiovascular drugs (e.g. clonidine), and in particular selected from the group consisting of escitalopram for use in the treatment of depression, pramipexole for use in the treatment of Parkinson's disease, buprenorphine for use in the treatment of opioid addiction or chronic pain, clonidine for use in the treatment of postmenopausal complaints or hypertension, gonadorelin for use in the treatment of the syndrome of Kallmann, insulin for use in the treatment of diabetes, nicotine for use in the support of smoking cessation or in the treatment of mild cognitive impairment, a progestogen for use in contraception, an SPRM for use in contraception, or analogs, homologs or agonists thereof or related compounds from the same chemical class and having the same pharmaceutical effect.
